# EUROPEAN PATENT SPECIFICATION

(11) **EP 2 803 371 B1**
(45) Date of publication and mention of the grant of the patent: **30.10.2019**
(21) Application number: 13735626.7
(22) Date of filing: 11.01.2013
(51) Int. Cl.: A61L 27/24, A61K 8/65, A61K 38/17, A61P 7/04, A61P 17/02, A61P 19/02, A61P 35/00, A61P 37/02, A61P 43/00, A61K 47/42, A61K 9/70, A61L 31/04, A61L 31/14, A61Q 19/08, A61K 8/02

(54) **COLLAGEN STRUCTURE, AND METHOD FOR PRODUCING COLLAGEN STRUCTURE**
KOLLAGENSTRUKTUR UND VERFAHREN ZUR HERSTELLUNG DER KOLLAGENSTRUKTUR
STRUCTURE EN COLLAGÈNE, ET SON PROCÉDÉ DE PRODUCTION

(30) Priority: 12.01.2012 JP 2012003883
(43) Date of publication of application: 19.11.2014
(73) Proprietor: Nippi Incorporated, Tokyo, 120-8601 (JP)
(72) Inventor: OGURA, Takayuki, Tokyo 120-8601 (JP); TANAKA, Keisuke, Tokyo 120-8601 (JP); OHBA, Yasuhiro, Tokyo 120-8601 (JP); HATTORI, Shunji, Tokyo 120-8601 (JP)
(74) Representative: Hering, Hartmut
(86) International application number: PCT/JP2013/050484
(87) International publication number: WO 2013/105665

(56) References cited:
- JP-A- 2010 172 247
- JP-A- 2010 273 847
- JP-B2- H07 100
- JP-B2- 2 820 209
- JP-B2- 3 221 690
- JP-B2- 4 064 435
- JP-B2- 4 251 665
- JP-B2- 4 671 365
- JP-B2- 4 681 214
- JP-B2- H0 622 579
- YUNOKI S ET AL: "Development of collagen condensation method to improve mechanical strength of tissue engineering scaffolds", MATERIALS CHARACTERIZATION, ELSEVIER, NEW YORK, NY, US, vol. 61, no. 9, 1 September 2010 (2010-09-01), pages 907-911, XP027132147, ISSN: 1044-5803 [retrieved on 2010-05-23]

## Description

### Technical Field

The present disclosure relates to a collagen structure comprising collagen fibers and a method of producing the collagen structure.

### Background Art

Collagen is a principal protein that constitutes skins, tendons, bones and the like of, for example, fish, pigs and cows. Since collagen is highly homologous among animals, it has a low antigenicity and is excellent in its biocompatibility and histocompatibility. Thus, collagen has excellent properties as a medical material. As artificial materials and the like that are capable of stably providing an implant tissue and avoiding immunorejection in the case of some sort of abnormality in a biological tissue, various members utilizing collagen as a raw material have been developed.

For example, there has been disclosed a cell-invasive medical material in which modified collagen having a helix content of 0 to 80% is bound to or coated on a carrier made of a synthetic resin or the like (Patent Literature 1). Although collagen has excellent tissue affinity, it is degraded by collagenase in vivo. In this cell-invasive medical material, in order to avoid such degradation, collagen whose properties for remaining in the body are improved by a cross-linking treatment is used. It is described that, when implanted into a living body or coated on a wound surface, the cell-invasive medical material according to Patent Literature 1 shows resistance to catabolic enzymes in the body, retains necessary mechanical strength for a certain period of time, has good affinity to cells and tissues, and is likely to allow proliferating cells to readily migrate into the inside.

There has been also disclosed a technology of using, as an artificial skin, a cross-linked collagen sponge which is obtained by adjusting the pH of a diluted collagen solution with acetic acid, adding glutaraldehyde thereto and then freeze-drying the resulting solution (Patent Literature 2). A collagen sponge implanted into an affected part such as a bum is known to provide numerous pores suitable for fibroblast proliferation because of its porous structure, help the fibroblast proliferation and thereby facilitate the healing of the affected part; however, in the preparation of conventional collagen sponges, the step of foaming a collagen solution is complex. In Patent Literature 2, it is described that a collagen sponge can be prepared without foaming a collagen solution.

In addition, there has been disclosed a collagen sponge comprising a microporous collagen hydrogel (Patent Literature 3). The invention of Patent Literature 3 is characterized in that a collagen sponge prepared in advance is impregnated with an aqueous solution of a hydrophilic organic solvent and then dried by a freeze-drying treatment. Collagen sponges can be used as an artificial skin, a wound-covering material or the like; however, conventional collagen sponges are stored being immersed in a solution and this is likely to cause deterioration of collagen. On the other hand, collagen sponges undergo contraction when they are stored in a dry state. The invention of Patent Literature 3 was made in view of these points. In examples thereof, a porcine tendon-derived atelocollagen having a concentration of 0.3% was homogenized on ice, frozen in a square molding frame and then freeze-dried under vacuum and further heat-dried under vacuum to be cross-linked, followed by immersion in a glutaraldehyde solution for further cross-linking. It is described that, by impregnating the collagen sponge prepared in this manner with an aqueous solution of a hydrophilic organic solvent and subsequently freeze-drying it at a temperature of -80°C or lower where contraction hardly occurs in general, the cracking of the resulting dry article can be reduced.

Further, there has been also disclosed a technology of producing a collagen structure by molding a collagen solution into a tubular or sheet form while concentrating the collagen solution (Patent Literature 4). In this technology, a circular collagen structure is formed by bringing a collagen solution into contact with a thickening agent such as polyethylene glycol via a permeable member so as to concentrate the collagen solution to a collagen concentration of 50 to 100 mg/ml and subsequently molding the concentrated solution into a circular form.

Still further, there has been disclosed a collagen gel comprising collagen fibers that are cross-linked by bringing a collagen solution not subjected to fiber formation into contact with an aqueous salt solution having buffering capacity and a cross-linking agent simultaneously (Patent Literature 5). Collagen gels are effective as cell carriers, medical materials and the like; however, they have poor thermal stability and their gel strength may not be satisfactory. In a conventional cross-linking method where a collagen gel is brought into contact with a protein cross-linking agent, although cross-linking takes place on the surfaces of collagen fibers, since the cross-linking agent does not infiltrate into the central part of the gel, the thermal stability of the gel is not sufficiently improved. According to Patent Literature 5, by allowing cross-linking reaction to take place between fibers in the middle of collagen fiber formation, the mechanical strength and thermal stability of the resulting collagen gel can be improved by the cross-linking and fiber formation.

Yet still further, there has been disclosed a collagen material comprising a laminate in which a collagen ultra-fine fibrous nonwoven fabric-like multilayer structure is sandwiched between non-fibrous collagen layers (Patent Literature 6). The invention of Patent Literature 6 was made in view of such problems that medical materials in which collagen is combined with a synthetic polymer material such as nylon may cause granulation, inflammation and/or the like that is attributed to the synthetic polymer material; and that cross-linked collagens using glutaraldehyde or epoxy pose a problem of toxicity caused by the cross-linking agent.

Furthermore, there has been disclosed a collagen implant having a density of about 0.01 to 0.3 g/cm³ (Patent Literature 7). This collagen implant is produced by: adding an alkali to an acidic aqueous solution of atelocollagen to allow collagen to be precipitated; preparing a dispersion by dissolving the resulting precipitates; casting the dispersion at a desired thickness; flash-freezing the thus casted dispersion to form a collagen matrix; and then compressing the collagen matrix to a thickness of about 1 to 20 mm. It is described that at least 80% of pores of this collagen implant have a diameter of 35 to 282 µm.

Moreover, there have been disclosed methods of producing a high-density cultured tissue which comprise performing circulation culture of a cell culture solution containing collagen and animal cells so as to allow the collagen and animal cells to be accumulated at a high density (Patent Literatures 8 and 9). According to these methods disclosed in Patent Literatures 8 and 9, an artificial tissue in which collagen and animal cells are accumulated at a high density can be quickly produced with simple operations.

### Citation List

### Patent Literature

Patent Literature 1: Examined Japanese Patent Application Publication No. H06-022579
Patent Literature 2: Japanese Patent No. 4681214
Patent Literature 3: Examined Japanese Patent Application Publication No. H07-000100
Patent Literature 4: Japanese Patent No. 3221690
Patent Literature 5: Japanese Patent No. 4064435
Patent Literature 6: Japanese Patent No. 4251665
Patent Literature 7: Japanese Patent No. 2820209
Patent Literature 8: Japanese Patent No. 4671365
Patent Literature 9: Unexamined Japanese Patent Application Kokai Publication No. 2010-172247

### Summary of Invention

### Technical Problem

In vivo, collagen exists extracellularly in a fibrous form and constitutes a variety of tissues at high concentrations of 25% in skin, 32% in tendons, 16% in cartilage, 23% in bone and 18% in dentin, per unit wet weight. In vivo collagen has a structure in which three polypeptide chains are twisted together into a triple helix and forms tropocollagens having a length of about 300 nm and a thickness about 1.5 nm, which associate with each other in a slightly staggered manner to form a thick and long fiber called "collagen microfibril". The bone matrix and cartilage matrix are constituted by the collagen microfibrils. Further, a plurality of the above-described collagen microfibrils associate with each other to form a large and strong fiber called "collagen fiber". Collagen fibers have a thickness of several micrometers to several tens of micrometers and constitute the skin dermis, tendons and the like. In this manner, collagen molecules form collagen fibers suitable for tissues through association, thereby exerting a wide variety of functions.

However, those collagen materials that are disclosed in the above-described Patent Literatures 1 to 3, 6 and 7 are all prepared using a collagen solution having a collagen concentration lower than the in vivo collagen concentration; therefore, in the resulting products, the collagen concentration is low or thick and long collagen fibers are not formed, so that these collagen materials cannot be tissue-equivalent materials. For instance, in Example 1 of Patent Literature 1, while stirring 0.3-w/v% atelocollagen solution, 0.3-w/v% denature atelocollagen solution is added thereto, and the resulting solution is subsequently subjected to rapid freezing and freeze-drying. In this collagen solution, since collagen molecules are discretely dissolved, no thick and long collagen fiber is formed, so that the dry article obtained by freeze-drying this collagen solution is not constituted by collagen fibers.

Further, in Example 3 of Patent Literature 2, glutaraldehyde is added to a solution having a collagen concentration of 3 mg/ml to a final glutaraldehyde concentration of 0.05 mM; 50 g of the resulting glutaraldehyde-containing diluted collagen solution is poured into a stainless-steel frame for freeze-drying (11 cm × 8.5 cm); the stainless-steel frame is cooled to -40°C to freeze the collagen foam solution; and the thus frozen collagen foam solution is then freeze-dried under reduced pressure (0.01 mmHg) at 30°C for 24 hours. Since collagen molecules are discretely dissolved in the collagen foam solution, similarly to Patent Literature 1, it is believed that no thick and long collagen fiber is formed.

Moreover, in Example 1 of Patent Literature 3, porcine tendon-derived atelocollagen having a concentration of 0.3% and pH of 3.0 is homogenized on ice and then frozen in a square frame, followed by freeze-drying under vacuum; therefore, similarly to Patent Literature 1, no thick and long collagen fiber is formed.

Furthermore, in Example 1 of Patent Literature 6, 1-wt% collagen solution is poured into a Petri dish to form a collagen solution layer, which is frozen at -20°C for 24 hours, freeze-dried at -80°C for 24 hours and then compressed to form a non-fibrous collagen layer. This non-fibrous collagen layer is also not constituted by collagen fibers. Here, in Patent Literature 7 as well, in order to produce a collagen matrix, a collagen solution is vacuum-suctioned at -20°C for 24 hours and then dried for about 8 hours under vacuum so as to remove the remaining water content. Since collagen molecules are discretely dissolved in this collagen solution, no thick and long collagen fiber is formed, so that the resulting collagen matrix is also not constituted by collagen fibers.

Meanwhile, since collagen is swollen with a small amount of water, it is not easy to produce dry collagen. Not only that, when dry collagen is obtained by freeze-drying a collagen solution, since the processing time is long and very large drying energy is required, it is also difficult to mold the resulting collagen into a desired shape. Therefore, it is desired to develop a production method which is capable of easily producing a collagen structure that is an artificial material having a high collagen concentration and can be molded into a thick article other than a film or a sheet.

Furthermore, those products that are disclosed in the above-described Patent Literatures 4 and 5 are both hydrates. Native collagen retaining a triple-helical structure has excellent moisture-retaining property and shows excellent cell adhesion activity; however, collagen dissolved in a solution has a low thermal denaturation temperature and is thus denatured even at normal temperature, so that it must be stored under refrigeration. Since these products of Patent Literatures 4 and 5 are both hydrates, they have poor thermal stability and are thus likely to be denatured by bacterial contamination or the like. In addition, since these products have a water content of 90 (w/w)% or higher, storage and transportation of these products are expensive. Therefore, it is desired to develop a collagen structure that has excellent biocompatibility and thermal stability as well as a low water content.

When an artificial medical material such as an artificial tissue or an artificial bone is used in regenerative medicine, the regenerative medicine material is applied to a defective site of dermis, bone, joint cartilage, tendon or the like to maintain a space where cells can migrate to promote regeneration. In order to allow such regeneration to take place smoothly, it is required that the medical material has excellent biocompatibility and is capable of maintaining cells and that the cells are able to moderately proliferate. The above-described cell-invasive medical material disclosed in Patent Literature 1 uses a synthetic resin such as polyester, polyurethane or vinyl chloride as a carrier; however, if the cell-invasive medical material could be constituted only by biological materials, inflammation and the like that are caused by the synthetic resin would be avoidable. Moreover, the above-described methods disclosed in Patent Literatures 8 and 9 are excellent in that they are capable of culturing animal cells in three dimensions; however, considering the convenience in storage and transportation, it is desired to develop a dry collagen structure.

In view of the above-described circumstances, an object of the present disclosure is to provide a collage structure which has a low water content and can be used in a wide range of medical applications and the like.

Another object of the present disclosure is to provide a method by which a collagen structure can be easily produced.

### Solution to Problem

The present inventors discovered that: when collagen fibers are generated by adding a neutral buffer to an acidic collagen solution and the resulting solution is gently stirred, association of collagen molecules is facilitated, so that thick and long collagen fibers are precipitated; by filtering this solution, crude collagen fibers having a collagen fiber concentration of 12 to 50 (w/v)% can be obtained; the crude collagen fibers, after being separated and molded into a prescribed shape, can be dried by freeze-drying or the like; the crude collagen fibers can also be dehydrated efficiently by dispersing them in a hydrophilic organic solvent; and a collagen structure can be produced by molding the separated collagen fibers into a prescribed shape and then air-drying the resultant, thereby completing the present disclosure.

That is, the present disclosure provides collagen structure, which is constituted by collagen fibers of 1 to 5 µm in average diameter; and has a water content of 0 to 15 (w/w)% and a collagen density of 50 to 800 mg/cm³.

The present disclosure also provides the collagen structure described above which further comprises at least one factor selected from the group consisting of cell chemotactic factors, growth factors, cell proliferation factors, blood coagulation factors and anticoagulant factors.

Further, the present disclosure provides the collagen structure described above which is used as an artificial medical material, a member for disease treatment, a cosmetic material or a cell culture material.

Still further, the present disclosure provides a method of producing a collagen structure, which comprises the steps of:
generating collagen fibers by neutralizing an acidic collagen solution;
forming crude collagen fibers having a collagen concentration of 12 to 50 (w/v)% by separating the collagen fibers from the solution containing the collagen fibers; molding the crude collagen fibers into a prescribed shape; and
drying a molded article obtained in the molding step.

Yet still further, the present disclosure provides the above-described method of producing a collagen structure, the method being characterized by further comprising the steps of, following the step of forming the crude collagen fibers: after dispersing the crude collagen fibers in a hydrophilic organic solvent, separating the collagen fibers from the hydrophilic organic solvent and dehydrating the thus separated collagen fibers; and molding the thus dehydrated collagen fibers.

Yet still further, the present disclosure provides the above-described method of producing a collagen structure, the method being characterized by further comprising the steps of, following the step of dehydrating the collagen fibers: subjecting the dehydrated collagen fibers to a cross-linking treatment and/or a chemical treatment; and drying the thus treated collagen fibers. Notwithsatnding the above disclosures, the invention is defined by the appended claims. According to the invention, a collagen structure is provided, which is constituted by collagen fibers of 1 to 5 µm in average diameter and 1 to 10 mm in average length which are measured for 20 fibers observed in a stereoscopic micrograph; and has a water content of 0 to 15 (w/w) % which is defined as percentage of an amount of water with respect to a mass of a collagen structure after heating at 120°C for 2 hours, whereby the change in the mass before and after the heating is determined as the amount of water, and the water content is defined as the percentage (%) of this amount with respect to the mass of collagen; and a collagen density of 50 to 800 mg/cm3, whereby a test piece with a defined thickness is dissolved in acetic acid and the collagen concentration is measured by a micorburet method, form the volume and collagen concentration of the test piece, the amount of collagen per unit volume is calculated as the collagen density.

Another aspect of the invention is a method of producing a collagen structure, which comprises the steps of:generating collagen fibers having 1 to 100 µm in average diameter and 1 to 10 mm in average length which are measured for 20 fibers observed in a stereoscopic micrograph, by neutralizing and gentle stirring an acidic collagen solution to facilitate an association of collagen fibers;forming crude collagen fibers having a collagen concentration of 12 to 50 (w/v)% by separating the collagen fibers from the solution containing the collagen fibers;molding the crude collagen fibers into a prescribed shape; anddrying a molded article obtained in the molding step.

### Advantageous Effects of Invention

According to the present disclosure, a collagen structure is prepared by drying crude collagen fibers having a collagen concentration of 12 to 50 (w/v)% which is constituted by collagen fibers of 1 to 5 µm in average diameter and 1 to 10 mm in average length in a prescribed shape; therefore, the collagen structure is equivalent to an in vivo collagen tissue. In addition, since the collagen structure is prepared using collagen fibers formed by association of plural collagen molecules as raw material, the collagen structure has excellent mechanical strength as well.

The collagen structure of the present disclosure has a water content of 0 to 15 (w/w)%; therefore, it has excellent thermal stability and is thus capable of efficiently avoiding deterioration caused by bacteria and the like.

According to the collagen structure production method of the present disclosure, drying can be performed by air-drying; therefore, in addition to a sheet-form article, a three-dimensional article can also be easily produced.

### Brief Description of Drawings

FIG. 1 is an image showing the sheet-form collagen structure produced in Example 1;
FIG. 2 is a stereoscopic micrograph showing the crude collagen fibers formed in Example 1;
FIG. 3 is a scanning electron micrograph (SEM) showing the surface of the collagen structure prepared in Example 1;
FIG. 4 is a scanning electron micrograph (SEM) showing a cross-section of the collagen structure prepared in Example 1;
FIG. 5 is a graph showing the results of measuring the denaturation temperature of the collagen structure prepared in Example 1 and that of the collagen solution used in the preparation of the collagen structure, using a differential scanning calorimeter (DSC) at a heating rate of 2°C/minute;
FIG. 6 is an image taken by a fluorescence microscope after swelling the collagen structure obtained in Example 1 with DMEM/10% FBS, inoculating the collagen structure with Human Foreskin Fibroblast (HFF) cells at a cell density of 1.0 × 10⁴ cells/cm² and then, 20 hours later, staining the cells with calcein AM;
FIG. 7 is an image showing the block-form collagen structure prepared in Example 2;
FIG. 8 is a scanning electron micrograph (SEM) showing the dry material produced in Comparative Example 1 by drying a collagen gel prepared from a collagen solution having a collagen concentration of 0.2 (w/v)%;
FIG. 9 is an image taken by a fluorescence microscope after acclimating the collagen gel obtained in Comparative Example 1 with DMEM/10% FBS, inoculating the collagen gel with HFF cells at a cell density of 1.0 × 10⁴ cells/cm² and then, 20 hours later, staining the cells with calcein AM; and
FIG. 10. is a scanning electron micrograph showing the collagen sponge that was produced in Comparative Example 3 by freeze-drying 1 (w/v)% collagen solution.

### Description of Embodiments

The first embodiment of the present disclosure is a collagen structure, which is composed of collagen fibers of 1 to 5 µm in average diameter; and having a water content of 0 to 15 (w/w)% and a collagen density of 50 to 800 mg/cm³. Further, the second embodiment of the present disclosure is the collagen structure described above which is used as an artificial medical material, a member for disease treatment, a cosmetic material or a cell culture material. The present disclosure will now be described in detail.

### (1) Collagen structure

The term "collagen" used herein refers to a protein constituting dermis, ligaments, tendons, bones, cartilages and the like. A molecule in which three peptide chains of collagen protein are twisted together into a triple helix is called "collagen molecule". In the present disclosure, the term "collagen fiber" refers to an assembly of collagen microfibrils and the term "collagen microfibril" refers to an assembly of plural collagen molecules.

Conventionally, type I to type XXIX collagens are known, and the collagen used in the present disclosure may be any of these collagens or a newly discovered collagen. The majority of collagens contained in a living body are insoluble in water and, in the present disclosure, those collagens that are capable of forming collagen fibers can be widely used. For example, a "solubilized collagen", which is obtained by solubilizing collagen contained in a raw material such as skin or bone of an animal by an addition of an enzyme such as protease, can be used. It is noted here that biological materials such as animal skins and bones may also contain a trace amount of "soluble collagen" that is soluble in a neutral salt solution and/or an acidic solution, such soluble collagen can be used also in the present disclosure. The constituent amino acids in the above-described "solubilized collagen" and "soluble collagen" may also be modified in performing a chemical treatment.

Further, the collagen molecules constituting the collagen fiber may also be collagen derivatives. In the present disclosure, the term "collagen derivative" means the above-described collagen molecule whose constituent amino acid(s) is/are modified with other functional group. Examples of such "collagen derivative" include acylated collagens and esterified collagens. As the acylated collagens, for example, succinylated collagens, phthalated collagens and maleylated collagens can be mentioned. Examples of "collagen derivative" also include acylated collagens such as succinylated collagens, phthalated collagens and maleylated collagens, which are obtained by adjusting an atelocollagen solution extracted by an enzyme treatment to have a pH of 9 to 12 and then adding thereto an acid anhydride such as succinic anhydride, phthalic anhydride or maleic anhydride. Further, examples of the esterified collagens include, in addition to those solubilized collagens that are esterified, insoluble collagens that are esterified and then solubilized by an enzyme reaction or the like.

In the present disclosure, the term "collagen structure" refers to a solid material having a prescribed shape. Therefore, the term "collagen structure" does not encompass any fluid such as powder or granule. Examples of the prescribed shape include film-forms, sheet-forms, and block-forms such as those of a cylinder, a cone, a polygonal column and a sphere. The prescribed shape may be any shape as long as it can be maintained, or it may be an amorphous shape as well. Here, the term "film-form" refers to the form of a thin film having a thickness of less than 200 µm and the term "sheet-form" refers to the form of a film having a thickness of not less than 200 µm. Further, the term "block-form" refers to an aggregate of planar material having a thickness in the vertical direction.

The collagen structure of the present disclosure comprises collagen fibers having an average diameter of 1 to 5 µm in a dry state. As described above, in a collagen solution, collagen molecules having a triple-helical structure are discretely dissolved; therefore, when such a collagen solution is molded into a film form by air-drying or the like, a film is formed by the collagen molecules and assemblies thereof. Since the collagen molecules and assemblies thereof are thin and short and the gaps between the collagen molecules and between the assemblies are thus small, cells cannot pass through the gaps. Even if cells were cultured on such a film, the cells would be localized on the film surface, not being able to migrate into the film. In addition, since the film is constituted by thin and short collagen molecules and the like, the mechanical strength of the film is low. However, in the present disclosure, since a collagen structure is constituted by thick collagen fibers of 1 to 5 µm in average diameter that are obtained by further association of collagen microfibrils formed by association of collagen molecules having a triple-helical structure, the gaps between the collagen fibers are large, so that cells can freely pass therethrough. Thus, when the collagen structure of the present disclosure is loaded to a living body, cells migrate into the collagen structure. Besides, the fiber structure of such collagen is similar to that of collagen found in the connective tissues of a living body such as tendons and ligaments. Therefore, the mechanical strength of the collagen itself can be maintained at a high level.

The collagen fibers constituting the collagen structure of the present disclosure have, in a dry state, an average diameter of 1 to 5 µm, more preferably 2 to 3 µm. In this range, a collagen structure having excellent cell infiltration property can be obtained. In the collagen fibers that are formed by association of collagen molecules, when the average diameter of the collagen fibers is 1 to 5 µm, the average fiber length is generally 1 to 10 mm as long as the collagen fibers are not subjected to physical cutting or any other treatment after the formation. It is noted here that, in the present disclosure, the average diameter and the average fiber length of the above-described collagen fibers are defined as the values that are measured for a collagen structure in a dry state, that is, in a state of having a water content of 0 to 15 (w/w)%, by the respective methods described below in the section of Examples.

The collagen structure of the present disclosure has a water content of 0 to 15 (w/w)%, more preferably 0 to 10 (w/w)%. Since the collagen structure is a dry material having a low water content, it has excellent thermal stability and is capable of avoiding deterioration caused by bacterial contamination and the like. In addition, the collagen structure is different from powder and the like in that it is a molded article in the form of a film, sheet, block or the like; therefore, by molding the collagen structure into the shape of a defective part of a living body, the collagen structure can be easily attached or loaded to the living body. It is noted here that, in the present disclosure, the water content is defined as the value measured by the method described below in the section of Examples.

In the collagen structure of the present disclosure, when the water content is 0 to 15 (w/w)%, the collagen density is 50 to 800 mg/cm³, more preferably 110 to 600 mg/cm³, particularly preferably 120 to 400 mg/cm³. Collagen exists in an insoluble form in vivo, forming connective tissues at high concentration of 25 (w/v)% in the skin tissue and 32 (w/v)% in the tendon tissue. In order to extract collagen from an animal tissue, collagen is required to be solubilized but the resulting solubilized collagen is highly viscous. Therefore, it is difficult to prepare a highly concentrated collagen solution and there has been thus no high-density collagen structure. However, according to the present disclosure, a collagen structure having a collagen density of 50 to 800 mg/cm³, which is equivalent to the in vivo collagen density, can be provided, and this collagen structure can be used as a tissue-equivalent material. It is noted here that, in the present disclosure, the collagen density is defined as the value measured by the method described below in the section of Examples.

The collagen structure of the present disclosure has a porosity of 20 to 90%, more preferably 30 to 80%, particularly preferably 40 to 70%. Since the collagen structure is porous, it is quickly swollen when immersed in a solvent. It is noted here that, in the present disclosure, the porosity is defined as the value measured by the method described below in the section of Examples.

The collagen structure of the present disclosure is a porous structure which comprises collagen fibers and has an average pore size of 1 to 50 µm, more preferably 5 to 30 µm. The collagen structure of the present disclosure is constituted in such a manner that the above-described collagen fibers are folded and overlapped as in a nonwoven fabric. Accordingly, the pores serve as communicating pores that can be in communication with other pores. Therefore, cells entering the pores can migrate into the inside of the collagen structure through the communicating pores. It is noted here that, in the present disclosure, the "average pore size" is defined as the value measured by the method described below in the section of Examples.

The collagen structure of the present disclosure may also comprise at least one factor selected from the group consisting of cell chemotactic factors, growth factors, cell proliferation factors, blood coagulation factors and anticoagulant factors. By adding these components, the collagen structure can be imparted with efficacies such as wound healing, inhibition of tumor cell proliferation, immunoregulation, osteogenesis, hematopoietic regulation, hemostasis and anticoagulation.

Examples of the chemotactic factors include cytokines such as erythropoietin and interleukin 1 (IL-1); and chemokines such as interleukin 8 (IL-8), NAP-2 and MIP-2.

Further, examples of the growth factors include epidermal growth factors (EGFs), insulin-like growth factor (IGFs), transforming growth factors (TGFs), nerve growth factors (NGFs) and platelet-derived growth factors (PDGFs).

Examples of the proliferation factors include brain-derived neurotrophic factors (BDNFs), vascular endothelial growth factors (VEGFs), granulocyte colony-stimulating factors (G-CSFs), granulocyte-macrophage colony-stimulating factors (GM-CSFs), erythropoietin (EPO), thrombopoietin (TPO), basic fibroblast growth factors (bFGF and FGF2) and hepatocyte growth factors (HGFs).

Further, examples of the coagulation factors include fibrinogen/fibrin (Factor I), prothrombin/thrombin (Factor II) and tissue factors (Factor III, thromboplastin), and examples of the anticoagulant factors include heparin and antithrombin III.

These additives may be bound to the collagen structure by impregnation or the like, or may be bound to the collagen structure via a bonding means, and the additives can be selected as appropriate in accordance with the intended use. For example, the collagen structure, which is impregnated with a solution containing the above-described component(s) to allow the component(s) to adsorb to the collagen structure and subsequently dried, can be used as a member of a drug delivery system or the like because it slowly releases the above-described component(s) upon being loaded to a wound.

Examples of the binding means include polypeptide chains of collagen-binding domains, such as the collagen-binding domain of von Willebrand factor and that of collagenase. By binding a polypeptide chain of a collagen-binding domain to the above-described components in advance, the components can be stably bound to collagen fibers via the binding means.

The collagen structure of the present disclosure may also be formed by performing cross-linking within each collagen fiber or between collagen fibers. Since collagen is a biological constituent, it is degraded in vivo by collagenase or the like. Accordingly, in cases where the collagen structure is used as a bone material or the like at a site or in an application where biodegradation is desired to be avoided, a cross-linked structure is introduced. By introducing a cross-linked structure, biodegradation is inhibited, so that the mechanical strength can be improved. Such a cross-linked structure may be introduced only to the surface of the collagen structure, or may be introduced to the inside of the collagen structure as well.

The collagen structure of the present disclosure is molded into a film form, a sheet form or a block form. The block-form may be a columnar-form, a spherical form or a cone-form, or the collagen structure may be molded into an arbitrary shape. Particularly, the collagen structure may also be molded into a specific shape of a biological tissue. Examples of the specific shape include biological shapes of a crescent constituting a knee joint, a tympanic membrane, a finger, a nose, an ear and the like; and those shapes of certain cartilages. By subcutaneously embedding the collagen structure of the present disclosure or by filling a bone fracture site with the collagen structure as an artificial bone, the neighboring cells are allowed to proliferate and, by applying the collagen structure of the present disclosure as an artificial skin to form a boundary between inside and outside the body, invasion of bacteria and the like can be inhibited and the regenerative function can be facilitated. It is noted here the collagen structure of the present disclosure may also comprise other layer(s) laminated thereon.

A conventional collagen sponge may be compressed into the form of a sheet having a high collagen density. However, since such a collagen sponge is not constituted by collagen fibers, it cannot secure such a strength that can be provided by collagen fibers. The collagen structure of the present disclosure is formed in prescribed shape without any compression processing; therefore, it has excellent cell infiltration property and is capable of maintaining a strength provided by the collagen fibers even when it is used in a hydrated state.

### (2) Application

The collagen structure of the present disclosure can be used as an artificial medical material, a member for disease treatment, a cosmetic material, a cell culture materials or the like.

As an artificial medical material, the collagen structure of the present disclosure is capable of adapting to a defective part of dermis, bone, joint cartilage, tendon, ligament, blood vessel or the like so as to facilitate the maintenance of space, introduction of cells and the like. Such an artificial medical material can be applied to regenerative medicine. Further, the collagen structure that is in a film form and impregnated with a hemostatic agent can be coated over a bleeding site to be used as a hemostatic material.

As a member for disease treatment, the collagen structure of the present disclosure can be used in the treatment of, for example, eye injury, severe burn, skin-grafted site, decubitus ulcer, diabetic ulcer, surgical incision wound or keloid-forming wound.

As a cosmetic material, the collagen structure of the present disclosure can be used as a pack material by cutting the film-form or sheet-form collagen structure into a face shape and impregnating it with a cosmetic lotion or the like.

As a cell culture material, by using the collagen structure of the present disclosure as a three-dimensional cell culture medium, cells can be subcultured. Further, since the collagen structure of the present disclosure has excellent cell infiltration and cell immobilization properties, it can be also used as, for example, a substrate for drug permeability test. Examples of subject cells to which the collagen structure of the present disclosure can be applied include ES cells and iPS cells.

Further, as an application of artificial medical material, the collagen structure of the present disclosure can be used as a carrier of a drug delivery system. When the collagen structure bound with various components is applied or loaded to a living body, the collagen structure releases the drug components with time, functioning as a drug delivery system.

### (3) Method of Producing Collagen structure

The method of producing the above-described collagen structure is not particularly restricted. However, the collagen structure can be produced by performing the steps of: generating collagen fibers by neutralizing an acidic collagen solution; forming crude collagen fibers having a collagen concentration of 12 to 50 (w/v)% by separating the collagen fibers from a solution containing the collagen fiber; molding the crude collagen fibers into a prescribed shape; and drying a molded article obtained in the molding step. The collagen structure can also be produced by further performing the steps of, following the step of forming the crude collagen fibers: after dispersing the crude collagen fibers in a hydrophilic organic solvent, separating the collagen fibers from the hydrophilic organic solvent and dehydrating the thus separated collagen fibers; and molding and drying the thus dehydrated collagen fibers. Moreover, a cross-linked collagen structure can be produced by further performing the steps of, following the step of dehydrating the collagen fibers: subjecting the dehydrated collagen fibers to a cross-linking treatment and/or a chemical treatment; and drying the thus treated collagen fibers.

The collagen to be used in the present disclosure can be collected from a skin of an animal such as cow, pig, bird or fish or other collagen-containing tissue. In general collagen is contained in a large amount in animal connective tissues; however, when extracted by a heat treatment, collagen is thermally denatured and its unique triple-helical structure is broken, causing the collagen to be in a gelatinous state. In the present disclosure, a collagen having a triple-helical structure is used. As a method of extracting such a collagen, for example, a solubilization method in which a material such as animal bone or skin is subjected to an acid treatment and/or an enzyme treatment can be employed. Preferred examples of the material from which the collagen is extracted include dermis and tendons of cow, pig, chicken, ostrich, horse, fish and the like. It is preferred to use a tissue of a young animal, such as an embryo-derived tissue, since the yield is improved.

For preparation of a collagen solution to be treated with an enzyme, for example, a tissue obtained by grinding and defatting the dermal layer of a bovine skin can be used. After suspending this tissue in distilled water to a final collagen concentration of 0.5 to 5 (w/v)%, the pH of the resulting suspension is adjusted to 3.0 by adding thereto hydrochloric acid. Then, acid protease is added in an amount of one-hundredth of the collagen weight to perform a solubilization treatment at 25°C for 72 hours. After terminating the enzyme reaction, the thus obtained enzyme-solubilized collagen solution is subjected to salt precipitation, and the recovered salt precipitates are then dispersed in distilled water to a collagen concentration of 1 to 5 (w/v)% and uniformly dissolved with an addition of hydrochloric acid, thereby a collagen solution can be obtained.

The pH of the above-described acidic collagen solution is preferably 1.0 to 6.0, more preferably 3.0 to 4.0. When the pH is higher than this range, it may be difficult to form collagen fibers.

In the present disclosure, the above-described acidic collagen solution is neutralized. Regardless of whether the collagen solution is prepared by an enzyme treatment or an acid treatment, the collagen solution is acidic for dissolving collagen molecules therein. An alkaline or neutral buffer is added to such acidic collagen solution. As an alkaline solution, for example, a sodium hydroxide solution or a potassium hydroxide solution can be used. Further, as the neutral buffer, a buffer which shows buffering action in the vicinity of pH 7.0, such as a phosphate buffer which comprises phosphoric acid and sodium phosphate and has a pH of 7.0 to 9.5, a HEPES (2-[4-(2-hydroxyethyl)-1-piperazinyl]ethanesulfonic acid) buffer (pH: 6.8 to 8.2), a citrate-phosphate buffer (pH: 2.6 to 7.0), a 50 mM Tris buffer (pH: 7.4) or a 50 mM phosphoric acid (pH: 7.4), can be widely used. It is noted here that "neutral" pH may be any pH of 6.0 to 9.0.

The above-described alkaline solution and neutral buffer may also contain other salt and the like in such an amount that does not change the pH. Examples of such a salt include sodium chloride and potassium chloride. When the collagen solution is made isotonic to human body fluid by an addition of such a salt, collagen fibers in which collagen molecules are staggered by 67 nm in the same manner as in vivo collagen can be formed. Therefore, it is preferred that the salt be added in such an amount that allows the osmotic pressure of the collagen solution after the neutralization treatment to be isotonic to human body fluid.

In the present disclosure, the collagen solution after the neutralization treatment has a collagen concentration of 0.01 to 5 (w/v)%, more preferably 0.1 to 5 (w/v)%, particularly preferably 0.3 to 5 (w/v)%. When the collagen concentration is lower than 0.01 (w/v)%, the subsequent concentration process is not easily carried out. Meanwhile, since collagen is highly viscous, it is difficult to prepare a collagen solution having a concentration of higher than 5 (w/v)%.

In the present disclosure, after the above-described neutralization treatment, the resulting collagen solution is left to stand in a temperature range of 4 to 45°C, more preferably 30 to 37°C. In this temperature range, the collagen molecules dissolved in the collagen solution are allowed to associate with each other in the solution by the neutralization treatment, thereby forming a collagen gel.

In the present disclosure, the resulting collagen gel is subsequently stirred gently. By this gentle stirring, association of the collagen molecules constituting the collagen gel is facilitated and the moisture contained between the fibers is released while the structure of the collagen fibers is maintained, so that thick and long collagen fibers are precipitated in the solution. Therefore, the stirring may be performed at any level as long as association of the collagen molecules can be facilitated. When the collagen solution is vigorously stirred, the generated collagen fibers are physically broken into thin and short collagen fibers. The collagen fibers precipitated in the solution by gentle stirring have an average diameter of 1 to 100 µm and a length of 1 to 10 mm. It is noted here that, in the present disclosure, the average diameter and the average fiber length of collagen fibers precipitated out of a collagen solution are defined as the values of average diameter and average length that are measured for 20 fibers randomly selected from those fibers observed in a stereoscopic micrograph, respectively.

By filtering or centrifuging this solution in which the collagen fibers are precipitated, the collagen fibers can be separated and recovered. In the present disclosure, the collagen fibers that are separated from the collagen solution are referred to as "crude collagen fibers". Accordingly, the crude collagen fibers comprise collagen fibers and water as main components. When the concentration of the collagen fibers contained in the crude collagen fibers is less than 12 (w/v)%, by again performing centrifugation, filtration or the like, the crude collagen fibers are further concentrated to a collagen concentration of 12 to 50 (w/v)%, more preferably 15 to 40 (w/v)%, particularly preferably 18 to 30 (w/v)%.

In order to separate the crude collagen fibers having the above-described concentration by filtration, it is preferred to use a filter paper having a pore size of 1 µm to 1 mm, more preferably 10 µm to 100 µm. As long as the pore size is in the above-described range, a large amount of collagen fibers can be efficiently processed.

Meanwhile, crude collagen fibers can also be separated by centrifuging the above-described collagen solution. For example, the collagen solution is centrifuged at 10,000 to 20,000 rpm for 10 minutes to 1 hour. Here, in order to adjust the collagen concentration to the above-described range, centrifugation can be performed a plurality of times.

In the present disclosure, the thus recovered crude collagen fibers are molded into a prescribed shape. As for the shape of the molded crude collagen fibers, the crude collagen fibers can be molded into a film form, a sheet form or a variety of three-dimensional configurations. In cases where the collagen structure is used for filling a tissue, it may be molded into a shape that conforms to the part to be filled in the subject body.

For example, in cases where a collagen solution in which collagen fibers are precipitated is filtered to separate crude collagen fibers, by arranging a filter paper on a porous filter paper mount formed in the middle part of a funnel and then filtering the collagen solution through the filter paper, the crude collagen fibers can be deposited in a sheet or block form on the filter paper. Alternatively, using the filter paper mount deformed into a prescribed shape in advance as a mold, the crude collagen fibers may be deposited on the filter paper mount and molded into a prescribed shape. The above-described methods are examples of embodiment where the step of forming crude collagen fibers and the molding step are performed continuously. Also, the crude collagen fibers deposited on the filter paper may be molded by being filled into a mold having a prescribed shape.

The above-described molding methods can be applied in the same manner also in those cases where crude collagen fibers are formed by centrifugation. For example, using a centrifuge tube as a mold at the time of performing centrifugation, crude collagen fibers can be centrifuged and molded into a prescribed shape at the same time. This method is another example of embodiment where the step of forming crude collagen fibers and the molding step are performed continuously. Here, after the centrifugation process, the crude collagen fibers may also be molded by being filled into a mold having a prescribed shape.

Subsequently, the molded crude collagen fibers are dried. In the production method of the present disclosure, since crude collagen fibers having a collagen concentration of 12 to 50 (w/v)% are molded, a collagen structure can be produced by dehydrating and drying the molded crude collagen fibers by freeze-drying, air-drying, hot-air drying, vacuum suction and/or the like. Here, a collagen structure having the shape of a cylinder, a column or the like may be obtained in advance and this may be further shaped by scraping or the like. As for the extent of the drying, the molded crude collagen fibers are dried to a water content of 0 to 15 (w/w)%. This is because the molded crude collagen fibers have superior storage stability as compared to the collagen solution.

The collagen structure of the present disclosure may also be subjected to compression molding after the above-described drying step. The collagen fibers constituting the collagen structure of the present disclosure have an average diameter of 1 to 5 µm and a length of 1 to 10 mm. Such thick and long collagen fibers are deposited in a nonwoven fabric-like form and the cell infiltration property and the strength are thereby maintained; therefore, even when the collagen structure of the present disclosure is subjected to compression molding, the collagen concentration can be increased without reduction in the cell infiltration property or the strength. Such compression molding may also be performed in a step other than those performed after the drying, for example, at the time of molding the crude collagen fibers into a prescribed shape.

In the present disclosure, after the separation of crude collagen fibers, a hydrophilic organic solvent solution dispersing crude collagen fibers may be prepared by adding a hydrophilic organic solvent to the crude collagen fibers in an amount of 3 to 2,000 parts by mass, preferably 5 to 1,000 parts by mass, more preferably 10 to 100 parts by mass, particularly preferably 10 to 30 parts by mass, and then the crude collagen fibers may be separated by filtration and dehydrated. The crude collagen fibers used in the present disclosure has a collagen concentration of 12 to 50 (w/v)%, the concentration is higher than that of a conventional collagen solution. By dispersing the crude collagen fibers in a high-concentration hydrophilic organic solvent such as 100% ethanol, highly hydrophilic crude collagen fibers can be efficiently dehydrated. Such a hydrophilic organic solvent solution dispersing crude collagen fibers has a higher fluidity than the collagen solution, so that the filtration efficiency of solution as well as the drying efficiency of the molded crude collagen fibers can be both improved. Since the occurrence of clogging is inhibited during the filtration operation, a thick block-form collagen structure can be produced.

Dehydration of collagen by ethanol or the like is conventionally known and it has been a common practice to dehydrate collagen while gradually increasing the alcohol concentration. However, in the present disclosure, since the crude collagen fibers have a high collagen concentration of 12 to 50 (w/v)%, for example, even when ethanol is used, 100% ethanol can be used. Therefore, dehydration by a hydrophilic organic solvent can be performed simply and efficiently.

The hydrophilic organic solvent for dispersing crude collagen fibers may be any carbon-containing solvent as long as it is miscible with water, and examples thereof include alcohols, ketones, ethers, esters and polar aprotic solvents. Examples of the alcohols include monohydric alcohols having 1 to 6 carbon atoms, such as methanol, ethanol, isopropanol and t-butanol; and polyhydric alcohols such as ethylene glycol and propylene glycol. Examples of the ketones include acetone and methyl ethyl ketone. Further, examples of the ethers include glycol ethers such as diethyl ether, methyl ethyl ether, ethylene glycol monomethyl ether and diethylene glycol monobutyl ether; and cyclic ethers such as tetrahydrofuran and dioxane. Moreover, examples of the esters include ethyl acetate and ethyl lactate, and examples of the polar aprotic solvents include dimethyl sulfoxide (DMSO), dimethylformamide (DMF) and pyridine. Thereamong, examples of preferred solvents that are miscible with water at an arbitrary ratio include acetone, methanol, ethanol, isopropanol, acetonitrile, tetrahydrofuran, dimethyl sulfoxide and dimethylformamide. Among these preferred solvents, ethanol, acetone, diethyl ether, or a mixed solution thereof can be suitably used.

Here, the temperature of the hydrophilic organic solvent to be used is preferably not higher than 15°C. This is because collagen fibers are not denatured at such a temperature and the triple-helical structure of the collagen molecules can thus be maintained.

By filtration or the like of the hydrophilic organic solvent solution dispersing crude collagen fibers, the crude collagen fibers can be isolated from the hydrophilic organic solvent solution and, consequently, the crude collagen fibers can be dehydrated. At the time of filtering the hydrophilic organic solvent solution containing crude collagen fibers, by arranging a filter paper on a porous-filter-paper-mounting-part formed in the middle of a funnel and then filtering the above-described hydrophilic organic solvent solution dispersing the crude collagen fibers through the filter paper, the crude collagen fibers are deposited in a sheet form on the filter paper. By this, dehydration and molding of the crude collagen fibers can be performed continuously. Also, by increasing the amount of deposition, the crude collagen fibers can be molded into a block form. It is noted here that the dehydrated crude collagen fibers can also be molded using a prescribed mold.

Collagen is highly hydrophilic and thus not readily dried. Particularly, it is not easy to dry a three-dimensional collagen. However, in the present disclosure, since crude collagen fibers having the above-described collagen concentration are dehydrated using a hydrophilic organic solvent, a collagen structure which has a high collagen density and is capable of retaining a three-dimensional shape can be produced.

After being molded, the crude collagen fibers can be dried also by freeze-drying or air-drying, although the drying method is variable depending on the shape and the size thereof. Air-drying is inexpensive and it can inhibit thermal denaturation of the collagen fibers.

The collagen structure of the present disclosure may further comprise a cross-linked structure. By introducing a cross-linked structure, decomposition of the collagen structure after it is embedded in a living body can be inhibited. The cross-linking method can be selected as appropriate in accordance with the intended use. For example, a cross-linked structure can be introduced by bringing the collagen fibers or collagen structure into contact with an aldehyde such as formaldehyde or glutaraldehyde, xylose, glucose, mannose, galactose or the like. Alternatively, the collagen structure can be cross-linked by adding thereto a carbodiimide-based, epoxide-based and/or imidazole-based cross-linking agent(s). Further, the collagen structure can also be cross-linked by irradiating it with ultraviolet ray, γ-ray, electron beam or the like. It is noted here that, when collagen is naturally dried, a cross-linked structure is partially formed in some cases.

Regardless of the presence or absence of cross-linking, the collagen structure of the present disclosure may be bound with at least one factor selected from the group consisting of cell chemotactic factors, growth factors, cell proliferation factors, blood coagulation factors and anticoagulant factors. Such factor(s) may be bound by chemical bonding or by physical bonding such as adsorption or deposition.

The step of binding the factor(s) can be performed in any of the steps for producing a collagen structure. For example, in any one of the steps prior to the step of drying the crude collagen fibers, at least one factor selected from the group consisting of cell chemotactic factors, growth factors, cell proliferation factors, blood coagulation factors and anticoagulant factors can be bound to the collagen fibers. The step of binding the factor(s) can be selected as appropriate in accordance with the chemical properties and the like of the component(s) to be added. For example, a collagen structure can be produced by: adding the above-described factor(s) to crude collagen fibers; uniformly stirring the resulting mixture to physically bind the factor(s) to the crude collagen fibers; molding the crude collagen fibers into a prescribed shape; and then drying the resultant. Alternatively, a collagen structure can be produced by: dispersing crude collagen fibers in a hydrophilic organic solvent; filtering the solvent to dehydrate the crude collagen fibers; mixing the thus dehydrated crude collagen fibers with above-described component(s); and then drying the resulting mixture.

Further, after producing a collagen structure having a water content of 0 to 15 (w/w)%, the collagen structure may be impregnated with an aqueous solution of the above-described factor(s) and then dried again to a water content of 0 to 15 (w/w)%.

In order to chemically bind the above-described factor(s) to the collagen structure, the factor(s) which a collagen-binding means is formed in advance may be used. Examples of such a binding means include the polypeptide chain of the collagen-binding domain of von Willebrand factor and that of the collagen-binding domain of collagenase. For example, by binding a polypeptide chain of a collagen-binding domain to the above-described factor(s) and then impregnating the collagen structure with a solution of the factor(s) having such a binding means, the factor(s) is/are bound via the binding means. An amino acid sequence of a collagen-binding domain is capable of specifically bind to collagen in the same manner as a collagenase which is an enzyme whose substrate is collagen.

The collagen structure of the present disclosure is characterized in that it has a high collagen density and is molded into a desired shape. As the shape, a film form, a sheet form, a block form or the like can be selected in accordance with the intended use. Thin-layer molded articles such as film-form and sheet-form molded articles, as well as collagen sponges and tubular collagen structures have been available; however, there has been no block-form collagen structure having a high collagen concentration. This is because it is difficult to improve the collagen concentration prior to drying. In the present disclosure, particularly by dispersing crude collagen fibers in a hydrophilic organic solvent to dehydrate the crude collagen fibers, a large deposit of the crude collagen fibers can be simply formed and easily dried by air-drying or the like. Further, by filling the large deposit into a mold having a prescribed shape, it can be molded and a collagen structure having a complex shape can be produced.

### Examples

The present disclosure will now be concretely described by way of examples thereof; however, the present disclosure is not restricted thereto by any means.

### (Example 1)

### (1) Preparation of Collagen structure

A tissue, which was prepared by grinding the dermal layer of a porcine skin using a meat grinder or the like and then defatting and sufficiently washing the resultant, was used as a raw material. In a solubilized aqueous solution in which pepsin and acetic acid were mixed at final concentrations of 5 mg/ml and 50 mM, respectively, the raw material was suspended to a final collagen concentration of 4.5 (w/v)%, and the resulting suspension was subjected to an overnight solubilization treatment at 4°C. To the resulting enzyme-solubilized collagen solution obtained in the above-described manner, sodium chloride was added to a final concentration of 5 (w/v)% to perform salt precipitation, and the thus formed precipitates were recovered by centrifugation. The recovered salt precipitates were dispersed in distilled water to a collagen concentration of 3 (w/v)% and then uniformly dissolved by adjusting the pH to 3.0 with an addition of hydrochloric acid, thereby preparing a collagen solution.

To 2.5 ml of this collagen solution (temperature: 4°C), 47.5 ml of phosphate-buffered saline (pH: 7.5, temperature: 4°C) was added, and the resultant was left to stand at 37°C for 24 hours.

By this process, collagen molecules were allowed to associate with each other to form a gelatinous material and, when this gelatinous material was gently stirred, the association was facilitated, so that collagen fibers were formed and dispersed in the resulting solution. The dispersed fibers were filtered out by pouring the solution onto a nylon mesh having a pore size of 80 µm, thereby recovering crude collagen fibers on the mesh. The thus obtained crude collagen fibers had a collagen concentration of 20 (w/v)%.

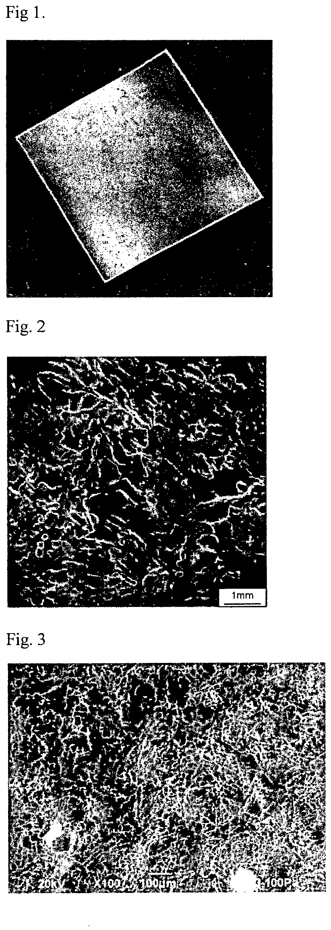

Then, the collagen fibers recovered on the mesh were freeze-dried to obtain a 0.2 mm-thick sheet-form collagen structure. The outer appearance of this collagen structure is shown in FIG. 1.

### (2) Water Content

The water content of the above-described collagen structure was measured to be 9.4 (w/w)% by the following method.

### (i) Method of Measuring Water Content

The mass of the collagen structure (w1) is measured. Then, after heating the collagen structure at 120°C for 2 hours to evaporate water, the mass of the resulting collagen structure (w2) is measured. The change in the mass before and after the heating (w1 - w2) is determined as the amount of water, and the water content is defined as the percentage (%) of this amount of water with respect to the mass of the collagen structure (w1).

### (3) Average Diameter and Average Fiber Length of Crude Collagen Fibers

The crude collagen fibers recovered on the nylon mesh were observed under a stereoscopic microscope. FIG. 2 is a stereoscopic micrograph thereof. Under the stereoscopic microscope, 20 crude collagen fibers were randomly selected, their diameters and lengths were measured, and the average values of the 20 fibers were calculated. The 20 crude collagen fibers had an average diameter of 1.15 µm and an average length of 4.09 mm. It is noted here that the shortest fiber length was 1.9 mm and the longest fiber length was 8.75 mm.

### (4) Scanning Electron Micrograph (Surface)

The surface fiber structure of the thus obtained collagen structure was observed under a scanning electron microscope (SEM). The result thereof is shown in FIG. 3.

### (5) Average Diameter and Pore Size of Collagen Fibers Constituting Collagen structure

For the above-described collagen structure, the average fiber diameter and the average pore size were measured in a dry state by the following methods. The results thereof are shown in Table 1. The collagen structure had an average pore size of 18.47 µm, meaning that the collagen structure had sufficient spaces for allowing cells of 5 to 7 µm in diameter to infiltrate.

### (i) Average Diameter of Collagen Fibers

From the collagen fibers observed under a scanning electron microscope (SEM), 20 fibers are randomly selected, and their diameters are measured. The average of the diameters measured for the 20 fibers is calculated as the average fiber diameter.

### (ii) Average Pore Size of Collagen Fibers

From the fibers observed under a scanning electron microscope (SEM), the diameters of randomly selected 20 fiber pores are measured. The average size of the 20 pores is calculated as the average pore size.

### (6) Scanning Electron Micrograph (Cross-section)

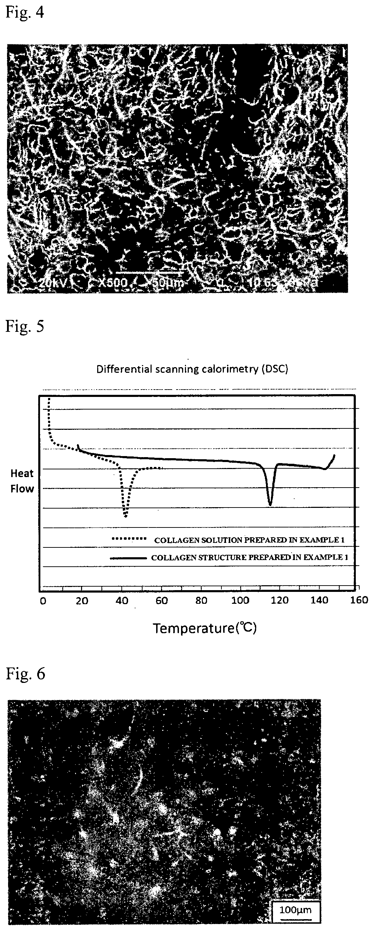

The fiber structure of a cross-section of the collagen structure was observed under a scanning electron microscope (SEM). The result thereof is shown in FIG. 4.

### (7) Denaturation Temperature

The denaturation temperature of the thus obtained collagen structure and that of the collagen solution used as a control were measured using a differential scanning calorimeter (DSC) at a heating rate of 2°C/minute. The results thereof are shown in FIG. 5 and Table 2. The collagen structure was observed to have a peak of denaturation temperature at 115.03°C, while the collagen solution had a denaturation temperature of 42.75°C. Therefore, it was revealed that the collagen structure had superior thermal stability as compared to the collagen solution.

### (8) Collagen Density and Porosity

The collagen density and the porosity were measured by the following methods. As a result, the collagen density was found to be 200 mg/cm³ and the porosity was found to be 40.9%.

### (i) Method of Measuring Collagen Density

The collagen structure is cut precisely into a size of 1-cm square to prepare a test piece. The thickness of this test piece is precisely measured using a thickness gauge so as to calculate the volume. Then, the test piece is dissolved in 5 ml of 5mM acetic acid solution and the collagen concentration is measured by a microburet method. From the volume and collagen concentration of the test piece, the amount of collagen per unit volume is calculated as the collagen density.

### (ii) Porosity

The porosity is measured by mercury intrusion porosimetry using Pascal 140 and 440 (manufactured by Carlo-Erba Instruments, Ltd.).

### (8) Cell Infiltration Property

The thus obtained collagen structure was swollen with DMEM/10% FBS and inoculated with HFF cells at a cell density of 1.0 × 10⁴ cells/cm². Then, 20 hours later, the cells were stained with calcein AM and observed under a fluorescence microscope. The result thereof is shown in FIG. 6.

### (Example 2)

### (1) Preparation of Collagen structure

A tissue, which was prepared by grinding the dermal layer of a porcine skin using a meat grinder or the like and then defatting and sufficiently washing the resultant, was used as a raw material. In a solubilized aqueous solution in which pepsin and acetic acid were mixed at final concentrations of 5 mg/ml and 50 mM, respectively, the raw material was suspended to a final collagen concentration of 4.5 (w/v)%, and the resulting suspension was subjected to an overnight solubilization treatment at 4°C. To the resulting enzyme-solubilized collagen solution obtained in the above-described manner, sodium chloride was added to a final concentration of 5 (w/v)% to perform salt precipitation, and the thus formed precipitates were recovered by centrifugation. The recovered salt precipitates were dispersed in distilled water to a collagen concentration of 3 (w/v)% and then uniformly dissolved by adjusting the pH to 3.0 with an addition of hydrochloric acid, thereby preparing a collagen solution. To 5 ml of this collagen solution (temperature: 4°C), 95 ml of phosphate-buffered saline (pH: 7.5, temperature: 4°C) was added, and the resultant was left to stand at 37°C for 24 hours. By this process, collagen molecules were allowed to associate with each other to form a gelatinous material

When this gelatinous material was gently stirred, the association was facilitated to form collagen fibers, which were dispersed and precipitated in the resulting solution. The precipitated fibers were recovered by 20-minute centrifugation at 17,500 rpm to obtain crude collagen fibers. The thus obtained crude collagen fibers had a collagen concentration of 20 (w/v)%.

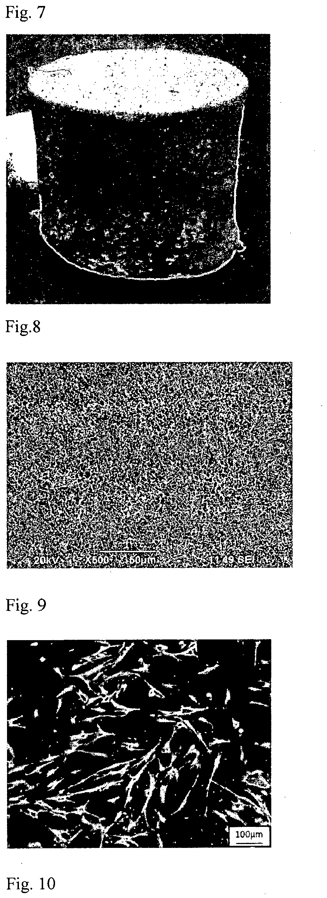

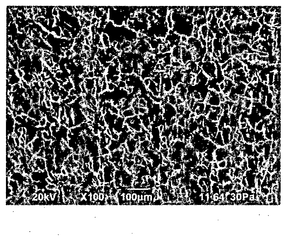

Thereafter, 0.75 g of the thus obtained crude collagen fibers was added to 10 g of 20°C ethanol and dispersed by gently stirring the resulting mixture for 10 minutes. The resulting dispersion was filtered to separate the crude collagen fibers. The thus recovered crude collagen fibers were filled into a columnar mold of 10 mm in diameter and 10 mm in height and then air-dried at room temperature to obtain a collagen structure. The thus obtained collagen structure is shown in FIG. 7.

### (2) Water Content, Collagen Density, Porosity, and Average Diameter of Collagen Fibers

For the thus obtained collagen structure, the water content, the collagen density, the porosity, and the average diameter of collagen fibers were measured in the same manner as in Example 1. As a result, it was found that this collagen structure had a water content of 6.7 (w/w)%, a collagen density of 127 mg/cm³ and a porosity of 76.6%. Further, the average diameter of the collagen fibers was 1.59 µm.

### (Comparative Example 1)

### (1) Preparation of Freeze-Dried Gel Material

The collagen solution obtained in Example 1 was diluted to a concentration of 0.4 (w/v)% by adding thereto distilled water and then mixed with an equivolume of 2× concentrated phosphate-buffered saline (pH: 7.5) at 4°C. The resulting mixture was gently poured on a cell culture plate and this plate was left to stand at 37°C for 24 hours to produce a gelatinous material.

The thus obtained gelatinous material was freeze-dried as it was, without isolating collagen fibers therefrom.

### (2) Water Content and Collagen Density

The water content and the collagen density were measured in the same manner as in Example 1. As a result, it was found that this freeze-dried material had a water content of 10 to 15 (w/w)% and a collagen density of 2.0 mg/cm³.

### (3) Average Diameter and Pore Size of Collagen Fibers

The average diameter and pore size of the collagen fibers constituting the freeze-dried material were measured in the same manner as in Example 1. The collagen fibers constituting this film were found to have an average diameter of 0.17 µm. It is noted here that the fiber length could not be measured since the collagen fibers were in contact with each other. The results are shown in Table 1.

### (4) Scanning Electron Micrograph of Gelatinous Material

The gelatinous material before being freeze-dried was observed under a scanning electron microscope (SEM). The result thereof is shown in FIG. 8.

### (5) Cell Infiltration Property

The gelatinous material before being freeze-dried was acclimated with DMEM/10% FBS and inoculated with HFF cells at a cell density of 1.0 × 10⁴ cells/cm² in the same manner as in Example 1. Then 20 hours later, the cells were stained with calcein AM and observed under a fluorescence microscope. The result thereof is shown in FIG. 9. In FIG. 6 of Example 1, a condition where the cells were three-dimensionally arranged was observed with both in-focus cells and out-of-focus cells existing at the same time; however, in FIG. 9, since the cells were in focus, it was observed that the cells existed two-dimensionally in a single plane.

### (Comparative Example 2)

The collagen solution obtained in Example 1 was adjusted to have a collagen concentration of 0.075 (w/v)% by adding thereto fivefold concentrated phosphate-buffered saline (pH: 7.5), and the resultant was stirred overnight at 37°C rather intensely (600 rpm) to form collagen fibers. The resulting collagen fiber-containing solution was stirred using a homogenizer to physically cut the collagen fibers or to inhibit binding of collagen molecules in the longitudinal direction. The collagen assembly contained in this solution had an average diameter of 1.13 µm and a length of 213 µm.

Then, the collagen assembly was recovered by 20-minute centrifugation at 17,500 rpm and centrifugation was repeated until a collagen concentration of 30 (w/v)% was attained. After dispersing the thus obtained precipitates in 20 times volume of ethanol, the resulting dispersion was filtered through a nylon mesh having a pore size of 80 µm to recover crude collagen assembly on the mesh. The thus obtained precipitates did not form a sheet and were in the form of powder.

### (Comparative Example 3)

The collagen solution obtained in Example 1 was diluted to a concentration of 0.8 (w/v)% by adding thereto distilled water and then freeze-dried as it was without neutralization, thereby preparing a collagen sponge. The thus obtained collagen sponge was porous and film-like structure and was not constituted by collagen fibers. FIG. 10 shows an electron micrograph of the collagen sponge. It is noted here that the collagen sponge had a collagen density of 8 mg/cm³.

**[Table 1]**

| | Example 1 | Comparative Example 1 |
|---|---|---|
| Average fiber diameter | 2.53 µm | 0.17 µm |
| Average pore size | 18.47 µm | 1.15 µm |

**[Table 2]**

| | Example 1 | |
|---|---|---|
| | Collagen solution | Collagen structure |
| Denaturation temperature | 42.75°C | 115.03°C |
| Amount of denaturation heat | 48.13 mJ/mg | 35.55 mJ/mg |

The present disclosure is based on Japanese Patent Application No. 2012-003883, which was filed on January 12, 2012.

### Industrial Applicability

The collagen structure according to the present disclosure is a dry material having a high collagen density. The collagen structure according to the present disclosure is useful since it also has a high thermal stability and can thus be used as a tissue-equivalent material in regenerative medicine.

## Claims

1. A collagen structure, which is constituted by collagen fibers of 1 to 5 µm in average diameter and 1 to 10 mm in average length which are measured for 20 fibers observed in a stereoscopic micrograph; and
has a water content of 0 to 15 (w/w) % which is defined as percentage of an amount of water with respect to a mass of a collagen structure after heating at 120°C for 2 hours, whereby the change in the mass before and after the heating is determined as the amount of water, and the water content is defined as the percentage (%) of this amount with respect to the mass of collagen; and a collagen density of 50 to 800 mg/cm³, whereby a test piece with a defined thickness is dissolved in acetic acid and the collagen concentration is measured by a micorburet method, form the volume and collagen concentration of the test piece, the amount of collagen per unit volume is calculated as the collagen density.

2. The collagen structure according to Claim 1, which has an average pore size of 1 to 50 µm measured for 20 pores in the collagen structure by a scanning electron microscope.

3. The collagen structure according to Claim 1 or 2, further comprising at least one factor selected from the group consisting of cell chemotactic factors, growth factors, cell proliferation factors, blood coagulation factors and anticoagulant factors.

4. The collagen structure according to any Claims 1 to 3, which is used as an artificial medical material, a member for disease treatment, a cosmetic material or a cell culture material.

5. A method of producing a collagen structure, which comprises the steps of:
generating collagen fibers having 1 to 100 µm in average diameter and 1 to 10 mm in average length which are measured for 20 fibers observed in a stereoscopic micrograph, by neutralizing and gentle stirring an acidic collagen solution to facilitate an association of collagen fibers;
forming crude collagen fibers having a collagen concentration of 12 to 50 (w/v)% by separating the collagen fibers from the solution containing the collagen fibers;
molding the crude collagen fibers into a prescribed shape; and
drying a molded article obtained in the molding step.

6. The method of producing a collagen structure according to Claim 5, further comprising the steps of, following the step of forming the crude collagen fibers: after dispersing the crude collagen fibers in a hydrophilic organic solvent, separating the collagen fibers from the hydrophilic organic solvent and dehydrating the thus separated collagen fibers; and molding the thus dehydrated collagen fibers.

7. The method of producing a collagen structure according to Claim 6, further comprising the steps of, following the step of dehydrating the collagen fibers:
subjecting the dehydrated collagen fibers to a cross-linking treatment and/or a chemical treatment; and
drying the thus treated collagen fibers.

## Patentansprüche

1. Kollagenstruktur, die durch Kollagenfasern mit einem mittleren Durchmesser von 1 bis 5 m und einer mittleren Länge 1 bis 10 mm erzeugt wird, die für 20 Fasern aufgezeichnet in einem Stereomikrobild gemessen werden; und
einen Wassergehalt von 0 bis 15 Gewichts-(w/w) %, der als Gehalt einer Menge Wasser in Bezug auf eine Masse einer Kollagenstruktur nach Erhitzen auf 120°C für 2 Stunden definiert wird, wobei die Änderung der Masse vor und nach dem Erhitzen als die Wassermenge bestimmt wird und der Wassergehalt ist definiert als Gewichtsprozent dieser Menge in Bezug auf die Kollagenmasse; und eine Kollagendichte von 50 bis 800 mg/cm³, wobei ein Probestück mit einer definierten Dicke in Essigsäure gelöst wird und die Kollegenkonzentration mit einem Mikrobüretteverfahren gemessen wird, aus dem Volumen und der Kollegenkonzentration des Probestückes die Menge Kollegen pro Volumeneinheit als Kollegendichte berechnet wird.

2. Kollegenstruktur nach Anspruch 1, die eine mittleren Porengröße von 1 bis 50 µm, gemessen für 20 Poren in der Kollegenstruktur mit einem Rasterelektronenmikroskop, aufweist.

3. Kollagenstruktur nach einem der Ansprüche 1 oder 2, des weiteren umfassend wenigstens einen Faktor ausgewählt aus der Gruppe bestehend aus Zellchemotaktische Faktoren, Wachstumsfaktoren, Zellproliferationsfaktoren, Blutkoagulationsfaktoren und Antikoalugantfaktoren.

4. Kollegenstruktur nach einem der Ansprüche 1 bis 3, die als ein künstliches medizinisches Material, ein Teil zur Krankheitsbehandlung, ein kosmetisches Material oder ein Zellkulturmaterial verwendet wird.

5. Verfahren zur Herstellung einer Kollegenstruktur, umfassen die Schritte:
Erzeugung von Kollagenfasern mit einem mittleren Durchmesser von 1 bis 100 µm und einer mittleren Länge von 1 bis 10 mm, die für 20 Fasern aufgezeichnet in einem Stereomikrobild durch Neutralisation und leichtes Rühren einer sauren Kollagenlösung, um die Assoziierung der Kollagenfasern zu erleichtern, gemessen wird;
Bildung von Rohkollagenfasern mit einer Kollagenkonzentration von 12 bis 50 (w/v) % durch Trennen der Kollagenfasern von der Lösung, die die Kollagenfasern enthält;
Pressen der Rohkollagenfasern in eine vorbeschriebene Form; und
Trocknen des in dem Pressverfahren erhaltenen gepressten Artikels.

6. Verfahren nach Anspruch 5, des weiteren umfassend nach dem Schritt der Bildung der Rohkollagenfasern die Schritte:
nach dem Dispergieren des Rohkollagenfasern in einem hydrophilen organischen Lösungsmittels werden die Kollagenfasern von dem hydrophilen organischen Lösungsmittel getrennt und die so getrennten Kollagenfasern werden dehydratisiert; und
die so erhaltenen Kollagenfasern werden gepresst.

7. Verfahren zur Herstellung einer Kollagenstruktur nach Anspruch 6, des weiteren umfassend nach dem Wasserentzugsschritt der Kollagenfasern die Schritte:
Unterwerfen der dehydratisierten Kollagenfasern einer Quervernetzungsbehandlung und/oder einer chemischen Behandlung; und
Trocknen der so behandelten Kollagenfasern.

## Revendications

1. Structure de collagène qui est constituée par des fibres de collagène de 1 à 5 µm de diamètre moyen et de 1 à 10 mm de longueur moyenne mesurés pour 20 fibres observées avec un microscope stéréoscopique, et
a une teneur en eau de 0 à 15 (w/w) % qui est définie en tant que pourcentage de la quantité d'eau par rapport à la masse de la structure de collagène après chauffage à 120°C pendant 2 heures, la modification de masse avant et après le chauffage étant déterminée en tant que quantité d'eau et la teneur en eau étant définie comme étant le pourcentage (%) de cette quantité par rapport à la masse de collagène, et une densité de collagène de 50 à 800 mg/cm³, une pièce test ayant une épaisseur définie étant dissoute dans de l'acide acétique et la concentration en collagène étant mesurée par la méthode de microburette à partir du volume et de la concentration en collagène de la pièce test, la quantité de collagène par volume unitaire étant calculée en tant que densité du collagène.

2. Structure de collagène conforme à la revendication 1,
ayant une dimension de pores moyenne de 1 à 50 µm mesurée pour 20 pores dans la structure de collagène avec un microscope électronique à balayage.

3. Structure de collagène conforme à la revendication 1 ou 2,
renfermant en outre au moins un facteur choisi dans le groupe formé par des facteurs chimiotactiques cellulaires, des facteurs de croissance, des facteurs de prolifération cellulaire, des facteurs de coagulation du sang et des facteurs d'anticoagulation.

4. Structure de collagène conforme à l'une quelconque des revendications 1 à 3, utilisée en tant que matériau médical artificiel, qu'un élément de traitement d'affections, que matériau cosmétique ou que matériau de culture cellulaire.

5. Procédé d'obtention d'une structure de collagène comprenant des étapes consistant à :
former des fibres de collagène ayant 1 à 100 µm de diamètre moyen et 1 à 10 mm de longueur moyenne mesurés pour 20 fibres observées avec un microscope stéréoscopique, en neutralisant et en agitant doucement une solution acide de collagène pour faciliter l'association de fibres de collagène,
former des fibres de collagène brutes ayant une concentration en collagène de 12 à 50 (w/v)% en séparant les fibres de collagène de la solution renfermant les fibres de collagène,
mouler les fibres de collagène brutes selon une forme prescrite, et
sécher l'article moulé obtenu lors de l'étape de moulage.

6. Procédé d'obtention d'une structure de collagène conforme à la revendication 5,
comprenant en outre des étapes consistant à la suite à l'étape de formation des fibres de collagène brutes après dispersion des fibres de collagène brutes dans un solvant organique hydrophile, séparer les fibres de collagène du solvant organique hydrophile et déshydrater les fibres de collagène ainsi séparées, et
mouler les fibres de collagène ainsi déshydratés.

7. Procédé d'obtention d'une structure de collagène conforme à la revendication 6,
comprenant en outre les étapes suivantes consistant à la suite de l'étape de déshydratation des fibres de collagène, soumettre les fibres de collagène déshydratées à un traitement de réticulation et/ou à un traitement chimique, et
sécher les fibres de collagène ainsi traitées.
